(19) Europäisches Patentamt
European Patent Office
Office européen des brevets

(11) **EP 2 152 187 B1**

(12) **EUROPÄISCHE PATENTSCHRIFT**

(45) Veröffentlichungstag und Bekanntmachung des Hinweises auf die Patenterteilung:
**18.07.2012  Patentblatt 2012/29**

(21) Anmeldenummer: **08749240.1**

(22) Anmeldetag: **30.04.2008**

(51) Int Cl.:
*A61B 19/04* (2006.01)          *B29C 41/22* (2006.01)
*A41D 19/00* (2006.01)

(86) Internationale Anmeldenummer:
**PCT/EP2008/003491**

(87) Internationale Veröffentlichungsnummer:
**WO 2008/135213 (13.11.2008 Gazette 2008/46)**

(54) **BEREICHSWEISE MEHRLAGIGER MEDIZINISCHER HANDSCHUH**

PARTIALLY MULTILAYER MEDICAL GLOVE

GANT MÉDICAL PARTIELLEMENT MULTICOUCHE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **04.05.2007  DE 102007021014**

(43) Veröffentlichungstag der Anmeldung:
**17.02.2010  Patentblatt 2010/07**

(73) Patentinhaber: **Paul Hartmann AG
89522 Heidenheim (DE)**

(72) Erfinder: **REIZEL, Clemens
89081 Ulm (DE)**

(56) Entgegenhaltungen:
EP-A- 0 561 651          WO-A-2006/065854
BE-A- 654 896          DE-A1- 4 444 800
US-A- 4 696 065          US-A- 5 459 880
US-A1- 2002 133 864          US-A1- 2003 124 354

**Beschreibung**

Die Erfindung betrifft mehrlagige wegwerfbare medizinische Handschuhe aus polymerem Material und ein Verfahren zu Herstellung eines solchen mehrlagigen wegwerfbaren medizinischen Handschuhs.

Wegwerfbare medizinische Handschuhe umfassen für den einmaligen Gebrauch vorgesehene Handschuhe, die den Patienten und den Anwender vor Kontaminationen, insbesondere bei der Durchführung von medizinischen Untersuchungen, und auch beim Umgang mit kontaminierten medizinischen Materialien schützen sollen und für diagnostische oder therapeutische Zwecke eingesetzt werden. Auch Handschuhe für die Anwendung in der Chirurgie sind davon erfasst.

Medizinische Handschuhe sind damit Handschuhe, welche als Untersuchungshandschuhe und Operationshandschuhe bekannt sind. Operationshandschuhe sind dabei sterile, anatomisch geformte medizinische Handschuhe. Untersuchungs- und Pflegehandschuhe können steril oder unsteril sein, und können auch anatomisch geformt sein.

Die wesentliche Bestimmung des Gebrauchs von derartigen medizinischen Handschuhen liegt somit in deren Schutzfunktion für Patient und Anwender.

Das Bedürfnis des Anwenders nach einer erhöhten Schutzfunktion beim Tragen von Handschuhen ist bekannt, vor allem um möglicher Übertragung von Viren, wie beispielsweise Hepatitis oder HIV, oder sonstigen Krankheitserregern noch besser vorzubeugen.

So wird dieses Bedürfnis in der Praxis durch das Tragen von zwei Handschuhen übereinander, dem sogenannten "double gloving", gelöst, um somit einen erhöhten Schutz, wie verbesserte Stichfestigkeit, im Vergleich zu einer einzelnen Handschuhlage zu erreichen. Nachteilig hierbei ist, dass das Anziehen von zwei Handschuhen übereinander für den Anwender beschwerlich und umständlich ist, und dies auch eine genaue Positionierung der Handschuhe übereinander verlangt, um ein gleichmäßiges Tastgefühl beizubehalten.

Weiterhin besteht bei den Anwendern von Handschuhen auch das Bedürfnis, dass neben dem erhöhten Schutz, die Sensitivität des Tastgefühls so wenig wie möglich beeinträchtigt wird. Dazu ist bekannt, wie beispielsweise in der GB 2 270 618 beschrieben, Handschuhe aus einem mindestens zweilagigen polymeren Verbundmaterial bereitzustellen, wodurch das beim "double gloving" vom Anwender als nachteilig gefühlte dickere polymere Material aufgrund einer vergleichsweise geringeren Dicke des Verbundmaterials überwunden wird.

Es sind auch Handschuhe bekannt, welche bereichsweise eine erhöhte Schutzfunktion durch das Vorhandsein mehrerer Lagen aufweisen.

DE 102005009826 A1 beschreibt medizinische Handschuhe mit verbesserter Schutzfunktion für definierte Untersuchungsfinger mittels einer Verstärkung dieser Fingeraufnahmebereiche oder mittels einer Doppelwandigkeit des Fingeraufnahmebereiches durch einen gesonderten übergestülpten oder einen überstülpbar verbundenen Fingerling.

EP 0 561 651 A1 beschreibt einen für den Einsatz beim Anlegen von orthopädischen Gipsverbänden vorgesehenen Handschuh aus einem Latexmaterial mit einer bereichsweise, vorzugsweise im Finger- und Handflächenbereich aufgebrachten Gleitschicht aus Polyvinylalkohol, welche im Tragezustand des Handschuhs auf der Außenseite des Handschuhs vorgesehen ist. Durch während des Herstellungsverfahrens eingebrachte Gegenmaßnahmen, wie Drehung des Formwerkzeugs um 180° nach dem Eintauchen in das Koagulant- und/oder Latexbad bei der Herstellung der Latexlage und Einbringen von Verdickungsmittel in die Gleitschichtlösung wird eine durch Gravitation üblicherweise verursachte Verdickung der Latex- und der Gleitschicht-Schicht in Richtung Fingerbereich verhindert. Bei der Tauchabfolge wird zuerst die Handschuhgrundstruktur bildende Polymerlage gebildet, dann die bereichsweise darauf gebundene Gleitschicht. Die beim Abstreifen des Handschuhs vom Formwerkzeug verursachte Inversion des Handschuhs wird durch weiteres Wenden unter Bereitstellung der Gleitschicht auf der Außenseite des Handschuhs aufgehoben.

Aus US 5459880 ist ein bereichsweise mehrlagiger Handschuh, als Arbeits- und als Haushaltshandschuh einsetzbar, mit einem Verstärkungsbereich im Finger- und Handflächenbereich und mit der bereichsweisen Beschichtungslage auf der Außenseite des Handschuhs angeordnet, bekannt.

US 2002/0133864 A1 offenbart ein Tauchverfahren in mehreren Schritten zur Herstellung eines bereichsweise zweilagigen Handschuhs und einen daraus erhaltenen Handschuh, wobei die Zweilagigkeit im Finger- und Handflächenbereich ausgebildet ist. Es liegen dabei eine transparente den Handschuhgrundkörper bildende Lage und eine davon unterscheidbare opake Beschichtungslage vor. Zum Einstellen der gewünschten möglichst gleichmäßigen Dicke des finalen Artikels werden aufeinander folgende Tauchschritte durchgeführt, und zwar zuerst in eine erste Koagulantlösung über die volle Höhe und dann in eine zweite Koagulantlösung über nur die teilweise Höhe, wobei die erste Koagulantlösung eine höhere Konzentration aufweist. Anschließend wird durch Tauchen in ein Polymerbad über die volle Höhe die Handschuhgrundstruktur bildende transparente Polymerschicht hergestellt, gefolgt durch Tauchen in das entsprechende Polymerbad über die teilweise Höhe zur Ausbildung der bereichsweisen opaken Polymerschicht.

BE 654896 offenbart ein Verfahren zur Herstellung von einlagigen medizinischen Handschuhen aus polymerem Material, mit einem im Vergleich zum Finger-/Handflächenbereich dickeren Stulpenbereich. Der dickere Stulpenbereich wird durch den Einsatz von unterschiedlichen Koagulantkonzentrationen erzielt, mit der Tauchabfolge zuerst in ein Koagulantbad höherer Konzentration über die volle Tauchhöhe, nachfolgend in ein Koagulantbad geringerer Konzentration bzw. in ein Bad mit Verdünnungsmitteln in reduzierter Tauchhöhe, und abschließend in das Polymerbad über die volle Tauchhöhe.

WO 03/057444 A1 bzw. US 2003/0124354 A1 offenbart einen mehrlagigen Handschuh mit mindestens zwei Lagen in einer Verbundstruktur, wobei die Lagen eine unterschiedliche Erstreckung haben, was dem Anwender farbunterstützt vermittelt wird. Die der Grundstruktur des Handschuhs angelagerte weitere Lage, welche optional auch noch eine Funktion einer Anziehhilfe oder auch eine Schutzfunktion innehaben kann, ist dabei in dem für die Benutzung vorgesehenen Zustand nach innen gerichtet.

Bei Anwendern von medizinischen Handschuhen, insbesondere von Operationshandschuhen, besteht das Bedürfnis, den von der üblicherweise zusätzlich getragenen, mit langen Ärmeln ausgestatteten medizinischen Schutzbekleidung, wie bspw. Untersuchungskittel oder Operationsmantel, ab dem unteren Ärmelende als ungeschützt verbliebenen Bereich der oberen Extremitäten, was also der Hand entspricht, besonders zu schützen.

Der Stulpenbereich von medizinischen Handschuhen, insbesondere von Operationshandschuhen wird dabei von den Anwendern vorzugsweise oberhalb des Ärmels der Schutzbekleidung getragen, was beim Anziehen und Überziehen des Handschuhs eine Flexibilität des Stulpenbereichs erfordert.

Der Erfindung liegt die Aufgabe zu Grunde, einen medizinischen Handschuh mit einer verbesserten Schutzfunktion bei gleichzeitigem Verbleib von Tastgefühl, Tragegefühl und auch einem erleichterten Anziehen des Handschuhs bereitzustellen.

Eine weitere Aufgabe ist die Bereitstellung eines Verfahrens zur Herstellung eines wegwerfbaren mehrlagigen medizinischen Handschuhs mit einer verbesserten Schutzfunktion.

Diese Aufgabe wird erfindungsgemäß gelöst durch einen medizinischen Handschuh mit den Merkmalen gemäß Anspruch 1.

Zur Lösung dieser Aufgabe wird ebenso ein Verfahren zur Herstellung eines derartigen medizinischen Handschuhs mit den Verfahrenschritten gemäß Anspruch 13 vorgeschlagen.

Vorteilhafte Weiterbildungen von Handschuh und Verfahren ergeben sich aus den Unteransprüchen.

Im Rahmen dieser Erfindung ist die "innere Lage" eine Lage des Handschuhs, welche im für den Gebrauch vorgesehenen und im für den Anwender bereitgestellten Zustand und auch im angezogenen Zustand des Handschuhs eine dem Körper des Anwenders zugewandte Lage ist. "Innen" bzw. "Innenseite" bedeutet grundsätzlich die Orientierung in Richtung Körper des Anwenders.

Die "äußere Lage" ist eine Lage des Handschuhs, welche im für den Gebrauch vorgesehenen und im für den Anwender bereitgestellten Zustand und auch im angezogenen Zustand des Handschuhes eine vom Körper des Anwenders abgewandte Lage ist. "Außen" bzw. "Außenseite" bedeutet grundsätzlich eine vom Körper des Anwenders abgewandte Orientierung.

Die erste innere polymere Lage ist in dem vorderen Abschnitt mit der zweiten äußeren polymeren Lage beschichtet, so dass die erste und die zweite Lage dort eine polymere zweilagige Verbundstruktur bilden.

Mit dem erfindungsgemäßen Vorschlag ist die zur Verbesserung der Schutzfunktion im vorderen Abschnitt vorhandene zweite Lage unter Bildung eines Verstärkungsbereiches im Gebrauch nach außen orientiert, so dass also die den Handschuh verstärkende zweite Lage direkt in Richtung der möglichen Gefahreneinwirkung von außen angeordnet ist.

In einer Anordnung der weiteren Lage innerhalb der die Handschuhgrundstruktur bildenden Lage, so wie in WO 03/057444 A1 angeordnet, kann diese doch flexiblere, schwächere Lage der Handschuhgrundstruktur mechanisch leichter beschädigt werden, was den Anwender aufgrund des scheinbar nicht mehr schutzfähigen Handschuhs zu einem sofortigen Wechsel veranlasst.

Die zweite äußere Lage des erfindungsgemäßen Handschuhs bildet damit das vorrangige Schutzschild, mit der Funktion, mögliche Beschädigungen und damit die Gefahreneinwirkung bereits an ihrer äußeren Oberfläche bzw. über die Erstreckung ihrer Dicke abzufangen bzw. zu stoppen, unter Beibehaltung einer intakten ersten inneren Lage.

Diese zweite äußere Lage bildet zusammen mit der ersten inneren Lage einen Verstärkungsbereich, welcher den vorderen Abschnitt des Handschuhs bildet und begrenzt.

Der vordere Abschnitt enthält dabei vollständig den Fingerbereich und zumindest bereichsweise den Handflächenbereich.

Es erweist sich als besonders vorteilhaft, wenn der vordere Abschnitt den Fingerbereich und auch den Handflächenbereich vollständig enthält.

Unter "Fingerbereich" wird die Gesamtheit aller Bereiche des Handschuhs verstanden, die für die Aufnahme der fünf einzelnen Finger vorgesehen sind, beginnend von der Basis des jeweiligen Fingers bis zur Fingerkuppe.

Der "Handflächenbereich" umfasst den Bereich zwischen dem Fingerbereich, abgegrenzt durch die Basis der jeweiligen Finger, und dem Stulpenbereich. Der Stulpenbereich beginnt in dem Bereich, an dem der Handflächenbereich beim Übergang in den Stulpenbereich den geringsten Umfang aufweist.

Der "Handflächenbereich" des Handschuhs umfasst die Handschuhinnenfläche und den Handrücken.

Das geschlossene Ende des vorderen Abschnitts wird durch die Fingerkuppen der einzelnen Fingeraufnahmebereiche gebildet, das offene Ende des anschließenden hinteren Abschnitts wird durch die herkömmliche Anziehöffnung des Handschuhs im Stulpenbereich gebildet.

Der den vorderen Abschnitt bildende und begrenzende Verstärkungsbereich enthält damit den Fingerbereich vollständig und den Handflächenbereich zumindest bereichsweise, insbesondere bevorzugt den Handflächenbereich vollständig, und stellt damit einen optimalen Schutz für den während eines pflegenden, therapeutischen und/oder chirurgischen Einsatzes insbesondere durch mechanische Beschädigungen, wie bspw. Nadelstichen am stärksten gefährdeten Bereich der Hand dar. Zusätzlich ist diese bereichsweise Verstärkung verbunden mit dem Vorteil, dass der unverstärkt verbliebene Bereich des Handflächenbereichs und/oder insbesondere des Stulpenbereichs die beim Vorgang des Anziehens des Handschuhs vom Anwender als vorteilhaft empfundene größere Flexibilität gegenüber dem Verstärkungsbereich, und insbesondere der Bedarf einer größeren Flexibilität beim Überziehen der Handschuhstulpe über den Ärmel eines Kittels weiterhin bereitgestellt ist.

In weiterer vorteilhafter Ausführung weisen der vordere Abschnitt und der hintere Abschnitt zusätzlich zu der unterschiedlichen Erstreckung der ersten inneren Lage und der zweiten äußeren Lage weitere Unterschiede auf.

Durch diese Unterschiede kann eine definierte verbesserte Schutzfunktion in dem Verstärkungsbereich erreicht werden.

Vorzugsweise unterscheiden sich der vordere Abschnitt und der hintere Abschnitt in den polymeren Materialien und/oder in ihren physikalischen Eigenschaften und/oder in der Barrierewirkung und/oder in der Farbe und/oder Kombinationen davon.

Die physikalischen Eigenschaften sind dabei vorzugsweise entnommen aus der Gruppe Reißkraft, Reißfestigkeit, Reißdehnung, maximaler Kraftaufwand oder Spannung bei 300% Dehnung, maximaler Kraftaufwand oder Spannung bei 500% Dehnung, Durchstichfestigkeit.

Hinsichtlich der Barrierewirkung unterscheiden sich der vordere und der hintere Abschnitt vorzugsweise in der Durchlässigkeit gegenüber Chemikalien, wie beispielsweise 80% Ethanol.

Als polymere Materialien für die Herstellung von medizinischen Handschuhen werden für den Fachmann herkömmlich bekannte Materialien eingesetzt. Bekannt sind Naturkautschuk und/oder Synthesekautschuke oder Kombinationen davon. Die Synthesekautschuke sind vorzugsweise entnommen aus der Gruppe umfassend Isoprenkautschuk (synthetisches Polyisopren), Chloropren-Kautschuk (Polychloropren), Acrylnitril-Butadien-Kautschuk (Nitrilkautschuk), hydrierter Nitrilkautschuk, Styrol-Butadien-Kautschuk, S-EB-S (Styrol-Ethylen-Buten-Styrol) Block-Copolymere, S-I-S (Styrol-Isoprene-Styrol) Block-Copolymere, S-B-S (Styrol-Butadien-Styrol) Block-Copolymere, S-I (Styrol-Isopren) Block-copolymere, S-B (Styrol-Butadien) Block-Copolymere, Butadien-Polymere, Styrol-Butadien Polymere, Carboxylierte Styrol-Butadien-Polymere, Acrylnitril-Styrol-Butadien Polymere, und deren Derivate.

Im wegwerfbaren mehrlagigen medizinischen Handschuhs ist die Gesamtdicke im Stulpenbereich geringer ist als im Handflächenbereich und/oder geringer als im Fingerbereich, mit der Gesamtdicke gemessen gemäß ISO 10282: 2002 (E) an den darin definierten Messpunkten.

Durch den Dickenunterschied zwischen vorderem Abschnitt und hinterem Abschnitt erfüllt der medizinische Handschuh das Bedürfnis des Anwenders nach der Kombination aus Schutzfunktion für den mechanischen Gefahren besonders ausgesetzten Bereich der Hand, unter anderem gewährleistet durch die größere Dicke, und Flexibilität durch eine geringere Dicke zumindest im Stulpenbereich.

Im Rahmen dieser Erfindung ist "Dicke" bzw. "Gesamtdicke" immer bezogen auf die Dicke bzw. Gesamtdicke der Einzelwand des Handschuhs.

Die Vermessung der Dicke erfolgt grundsätzlich nach ISO 4648:1991, Methode A mit einem Dickenmessgerät bei einem Prüfdruck von 22 kPa +/- 5 kPa.

Die Dickenmessung gemäß ISO 10282:2002(E) und der darin zitierten ISO 4648, Methode A wird an einem intakten Handschuh über die übereinander liegenden Einzelwände, und damit als Dickenmessung über die doppelte Wand durchgeführt. Die Dicke der Einzelwand ergibt sich aus der Hälfte der gemessenen Doppelwanddicken.

Bei der Vermessung der Dicke nach ISO 10282:2002(E) werden

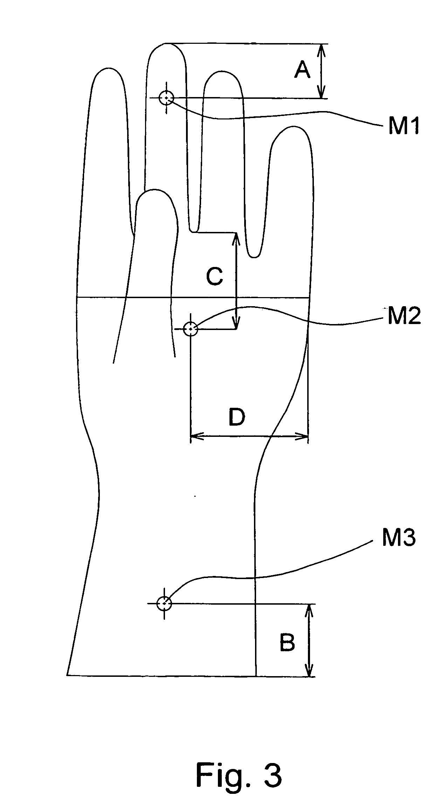

die entsprechenden Proben, also die medizinischen Handschuhe ohne weitere Vorbehandlung in einem flachausgelegten Zustand vermessen, und zwar bei einem Prüfdruck von 22 kPa +/- 5 kPa. Die Messungen werden dabei an definierten Messpunkten, wie in Figur 3 dargestellt, durchgeführt.

Die Distanzen sind dabei wie folgt:

A: 13 ± 3 mm; B: 25 ± 5 mm; C: 33 ± 7 mm; D: 48 ± 9 mm.
Messpunkt 1 liegt dabei innerhalb des Fingerbereichs,
Messpunkt 2 liegt dabei innerhalb des Handflächenbereichs und
Messpunkt 3 liegt dabei innerhalb des Stulpenbereichs.

In weiterer vorteilhafter Ausführungsform des wegwerfbaren mehrlagigen medizinischen Handschuhs verhält sich die Gesamtdicke im Stulpenbereich zur Gesamtdicke im Handflächenbereich in einem Verhältnis von höchstens 1: 1.2, mit der Gesamtdicke gemessen an den Messpunkten gemäß ISO 10282:2002(E). Es ist hierbei zu verstehen, dass die Gesamtdicke im Handflächenbereich höchstens um 20% größer ist als die Gesamtdicke im Stulpenbereich.

Vorteilhafterweise weist der Handschuh ausgehend vom geschlossenen Ende des vorderen Abschnitts über die Gesamtlänge L bis zum offenen Ende des hinteren Abschnitts eine Gesamtdicke mit einem abfallenden Dickenprofil auf.

Die Gesamtdicke des Handschuhs nimmt in dieser Richtung also ab.

In weiterer vorteilhafter Ausführung weist der Handschuh vom geschlossenen Ende des vorderen Abschnitts über die Gesamtlänge L bis zum offenen Ende des hinteren Abschnitts ein abfallendes Dickenprofil auf, mit einer Gesamtdicke von höchstens 0,40 mm, vorzugsweise höchstens 0,35 mm, weiter vorzugsweise höchstens 0,30 mm im vorderen Abschnitt und einer Gesamtdicke von mindestens 0,15 mm, vorzugsweise mindestens 0,18 mm, weiter vorzugsweise mindestens 0,20 mm im hinteren Abschnitt, mit der Dicke gemessen mittels eines Prüfgeräts gemäß ISO 4648:1991, Methode A, bei einem Prüfdruck von 22 kPa +/- 5 kPa.

Die erste innere Lage weist ausgehend vom geschlossenen Ende des vorderen Abschnitts in Längsrichtung zum offenen Ende des hinteren Abschnitts eine Dicke mit einem ansteigenden Dickenprofil auf.

Besonders bevorzugt weist die erste innere Lage dabei ausgehend vom geschlossenen Ende des vorderen Abschnitts in Längsrichtung zum offenen Ende des hinteren Abschnitts ein ansteigendes Dickenprofil auf mit einer Dicke im vorderen Abschnitt von mindestens 0,08 mm, vorzugsweise mindestens 0,10 mm, weiter vorzugsweise mindestens 0,15 mm und im hinteren Abschnitt von höchstens 0,30 mm, vorzugsweise höchstens 0,27 mm, weiter vorzugsweise höchstens 0,25 mm.

Weiter weist die zweite äußere Lage ausgehend vom geschlossenen Ende des vorderen Abschnitts in Längsrichtung innerhalb des vorderen Abschnitts eine Dicke mit einem abfallenden Dickenprofil auf.

Besonders bevorzugt weist die zweite äußere Lage dabei ausgehend vom geschlossenen Ende des vorderen Abschnitts in Längsrichtung innerhalb des vorderen Abschnitts ein abfallendes Dickenprofil auf ausgehend von einer Dicke von höchstens 0,25 mm, vorzugsweise höchstens 0,20 mm, weiter vorzugsweise höchstens 0,15 mm.

In einer vorteilhaften Ausführungsform hat der Handschuh im Stulpenbereich eine Gesamtdicke von 0,23 - 0,27 mm, im Handflächenbereich eine Gesamtdicke von 0,25 - 0,28 mm und im Fingerbereich eine Gesamtdicke von 0,28 - 0,34 mm, mit der Gesamtdicke jeweils gemessen an den Messpunkten gemäß ISO 10282:2002(E).

Ein vorteilhafter Unterschied zwischen dem vorderen und dem hinteren Abschnitt liegt in der physikalischen Eigenschaft der Reißkraft.

Die Reißkraft beschreibt die maximale Kraft, also die erste maximale Kraftspitze im Kräfteverlauf der Messung, welche benötigt wird um einen definierten Probenkörper zu zerreißen.

Im Falle von mehrlagigen Probenkörpern mit Materialien, welche unterschiedliche Eigenschaften, bspw. hinsichtlich Festigkeit, Elastizität und damit auch unterschiedliches Verhalten bei Zugprüfmessungen aufweisen, wird bei Messungen von Reißkraft, Reißdehnung, Reißfestigkeit stets der Messpunkt die ersten Reißens, also die erste vermessene Kraftspitze verstanden.

Vorteilhafterweise weist der medizinische Handschuh im hinteren Abschnitt eine größere Reißkraft auf als im vorderen Abschnitt.

Vorzugsweise ist die Reißkraft im hinteren Abschnitt mindestens um den Faktor 2, insbesondere mindestens um den Faktor 2,5, weiter insbesondere mindestens um den Faktor 3 größer als die Reißkraft im vorderen Abschnitt.

Die Reißkraft wird dabei mit standardisierten Methoden gemessen, wie mittels der Messmethode nach EN 455-2:2000.

Der hintere Abschnitt weist dabei vorteilhafterweise eine Reißkraft von mindestens 16 N, insbesondere mindestens 18 N, weiter insbesondere mindestens 24 N auf, gemessen nach EN 455-2:2000 als Reißkraft vor künstlicher Alterung.

Vorzugweise weist der hintere Abschnitt eine größere Reißdehnung auf als der vordere Abschnitt.

Die Reißdehnung wird als die am Messpunkt der Reißkraft maximal erreichte Dehnung(ausgedrückt in %) verstanden.

Diese sogenannte Reißdehnung wird hierbei innerhalb der standardisierten Methode nach DIN EN 455-2:2000 ermittelt.

Der nach DIN EN 455-2:2000 erforderliche Prüfkörper wird spannungslos über mindestens 16 h bei 23°C und 50% relativer Luftfeuchtigkeit klimatisiert. Anschließend wird der Prüfkörper in eine vertikale Zugprüfeinrichtung nach EN ISO 527-1 (1996) eingespannt, wobei der Abstand für die Einspannvorrichtung, also die Ausgangslänge L0 70 mm beträgt. Hierbei wird der Prüfling über seine gesamte Breite an einem Ende in die feststehende Klemmbacke fest eingespannt. An dem anderen Ende wird der Abschnitt über seine gesamte Breite in die bewegliche Klemmbacke der Zugprüfeinrichtung fest eingespannt und es wird eine Vorkraft von 0,12 N angelegt. Die bewegliche Klemmbacke wird mit einer Geschwindigkeit von 500 mm/min bewegt bis der Prüfkörper die maximale Kraft zum ersten Reißen erreicht hat. Anschließend wird die gedehnte Länge L1 des Abschnittes in mm gemessen.

Die Dehnung in % berechnet sich dann nach folgender Formel:

$$Dehnung[\%] = \frac{L1[mm] - L0[mm]}{L0[mm]} x100\%$$

Vorteilhafterweise ist die Reißdehnung im hinteren Abschnitt mindestens um den Faktor 1,5, insbesondere mindestens um den Faktor 1,7, weiter insbesondere mindestens um den Faktor 2 größer als die Reißdehnung im vorderen Abschnitt. Vorzugsweise weist der vordere Abschnitt eine Reißdehnung von 200 - 800%, insbesondere von 300 - 700%, weiter insbesondere von 400 - 600% auf. Vorzugsweise weist der hintere Abschnitt eine Reißdehnung von mindestens 800%, insbesondere von mindestens 900%, weiter insbesondere von mindestens 1000% auf.

Die Reißfestigkeit ausgedrückt in MPa bzw. $N/mm^2$ ist der Quotient aus der im Augenblick des Reißens des Prüfkörpers gemessenen Kraft (also die Reißkraft) und der Größe der Fläche des Anfangsquerschnittes des Prüfkörpers.

Vorteilhafterweise ist die Reißfestigkeit im hinteren Abschnitt größer als im vorderen Abschnitt. Insbesondere vorteilhaft ist die Reißfestigkeit im hinteren Abschnitt mindestens um den Faktor 2, insbesondere mindestens um den Faktor 2,5, weiter insbesondere mindestens um den Faktor 3 größer als die Reißfestigkeit im vorderen Abschnitt.

Durch die unterschiedliche Reißkraft, Reißdehnung bzw. Reißfestigkeit im vorderen und hinteren Abschnitt, also mit einer vergleichsweise höheren Reißkraft, Reißdehnung bzw. Reißfestigkeit im hinteren Abschnitt wird insbesondere dem Bedürfnis des Anwenders nach Flexibilität im Stulpenbereich Rechnung betragen. Zudem wird die Mindestanforderung an Reißkraft, Reißdehnung bzw. Reißfestigkeit im vorderen Abschnitt erfüllt, um ein zu schnelles Zerreißen des Handschuhs im vorderen Abschnitt, wie bei der Zugbelastung beim Anziehen des Handschuhs zu vermeiden.

Ein weiterer Parameter ist der maximale Kraftaufwand [N] oder die maximale Spannung [MPa], welche benötigt wird um eine definierte Dehnung des Materials, insbesondere eine Dehnung von 300%, weiter insbesondere eine Dehnung von 500% zu erreichen.

Die Messung des Kraftaufwandes für die Dehnung von 300% wird nach der standardisierten Methode ISO 10282:2002(E)durchgeführt.

Ein weiterer vorteilhafter Unterschied zwischen dem vorderen und dem hinteren Abschnitt liegt im Kraftaufwand bei 300% Dehnung.

Vorzugsweise weist der vordere Abschnitt bei 300% Dehnung eine höheren maximalen Kraftaufwand auf als der hintere Abschnitt, gemessen nach Methode ISO 10282:2002 (E) in Verbindung mit der darin zitierten ISO 37.

In weiterer vorteilhaften Ausführung weist der vordere Abschnitt bei 300% Dehnung einen maximalen Kraftaufwand auf, welcher mindestens um den Faktor 1,5, insbesondere um den Faktor 2,0, weiter insbesondere um den Faktor 2,5 größer ist als der maximale Kraftwand bei 300% Dehnung im hinteren Abschnitt.

Ein weiterer vorteilhafter Unterschied zwischen dem vorderen und dem hinteren Abschnitt liegt in der benötigten Spannung für eine Dehnung des Materials um 500%.

Vorzugsweise weist der vordere Abschnitt bei 500% Dehnung eine höhere maximale Spannung auf als der hintere Abschnitt, gemessen nach Methode ASTM D 3577-01a. In weiterer vorteilhaften Ausführung weist der vordere Abschnitt zum Erreichen einer Dehnung um 500% eine maximale Spannung auf, welche mindestens um den Faktor 1,5, insbesondere mindestens um den Faktor 1,7, weiter insbesondere mindestens um den Faktor 2,0 größer ist als die maximale Spannung für 500% Dehnung im hinteren Abschnitt.

In einer besonders vorteilhaften Ausführungsform weist der medizinische Handschuh eine Wirksamkeit gegenüber Durchstich, also eine Durchstichfestigkeit, auf.

Insbesondere vorteilhaft weist der vordere Abschnitt eine höhere Durchstichfestigkeit auf als der hintere Abschnitt.

Die Durchstichfestigkeit kann ausgedrückt werden als die Durchstichkraft nach EN 388:1994.

Besonders vorteilhaft hat der vordere Abschnitt eine Durchstichkraft mit mindestens der Leistungsstufe 1 gemäß EN 388:1994.

Das Prüfmuster wird dabei entsprechend der Norm EN 388:1994 mit der Außenseite in die Haltevorrichtung eingespannt.

Es wird das Prüfmuster aus dem Verstärkungsbereich aus dem Handflächenbereich entnommen, und entsprechend der Norm mit der zweiten äußeren Lage gegen den Stift des Prüfgerätes in die Haltevorrichtung eingespannt.

Die Durchstichfestigkeit kann auch gemessen werden nach der nachfolgend beschriebenen Methode, welche an die Methode ASTM D 3787-01 angelehnt ist:

Durchstichfestigkeit in Anlehnung an ASTM D 3787-01

Die ASTM D 3787-01 beschreibt die Bestimmung der Berstfähigkeit mit einem Kugel-Berstfestigkeitstester an Textil - oder Kunststoff-Flachmaterialien mit hoher Bruchdehnung.

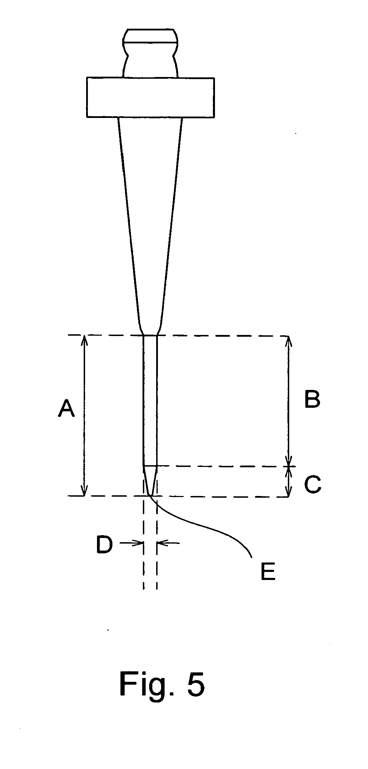

Anstelle der darin beschriebenen Durchstoßkugel wird eine Prüfspitze, wie in Figur 5 dargestellt, eingesetzt. Diese Prüfspitze umfasst dabei einen Nadelbereich mit der Gesamtlänge A von 50 mm. Diese Nadel hat über die Länge B von 40 mm einen gleichbleibenden Durchmesser D von 4 mm. Die Nadelspitze läuft über die Distanz C von 8 mm konisch zum Nadelspitzenpunkt E mit einem Durchmesser von 0.5 mm zu.

Die zu untersuchenden Handschuhe werden bis zur Gleichgewichtsfeuchte bei 23°C und bei 50% relativer Luftfeuchtigkeit klimatisiert. Die Dimension der aus den Handschuhen entnommenen Prüflinge ist dabei derart gewählt, dass diese mittels des Klemmrings mit einem Innendurchmesser von 45 mm plan und spannungsfrei in der Prüfvorrichtung nach ASTM D 3787-01 befestigt werden können. Bei Prüflingen, die aus dem vorderen Abschnitt des medizinischen Handschuhs entnommen sind, wird der Prüfling mit der zweiten äußeren Lage in Richtung der Prüfspitze eingespannt. Die Prüfspitze wird an einer Prüfmaschine nach EN ISO 527-1 befestigt. Bei einer Prüfgeschwindigkeit von 305 $\pm$ 12 mm/min wird die Prüfung bis zum Bersten des Prüflings fortgesetzt und der Kraftverlauf dabei registriert.

Insbesondere vorteilhaft weist der vordere Abschnitt eine größere Durchstichkraft auf, welche vorzugsweise mindestens um den Faktor 1,2 , insbesondere mindestens um den Faktor 1,3 größer ist als die Durchstichkraft im hinteren Abschnitt.

Durch die größere Durchstichfestigkeit im vorderen Abschnitt bietet der vorteilhafte medizinische Handschuh damit einen Verstärkungsbereich, welcher insbesondere bei Operationen, bei deren Arbeitsschritte mechanische Gefahrenquellen beispielsweise durch Spritzen, Spitzen von Absaugkanülen, spitze Ansatzstellen von Skalpellen gegeben sind, besonders geeignet ist.

Der Einsatz von verschiedenen polymeren Materialen in der ersten inneren und in der zweiten äußeren Lage ist vorteilhaft, da die Eigenschaften der polymeren Materialien gezielt für den hinteren und den vorderen Abschnitt, und damit für den Verstärkungsbereich eingesetzt werden können.

Die erste und/ oder die zweite Lage umfasst vorzugsweise polymere Materialien, welche entnommen sind aus der Gruppe Naturkautschuk und synthetischer Kautschuk, davon insbesondere synthetisches Polyisopren, Polychloropren, Acrylonitril-Butadien-Kautschuk und Derivate, oder Kombinationen dieser polymeren Materialien.

In einer bevorzugten Ausführungsform wird für die erste innere polymere Lage Naturkautschuk und für die zweite äußere polymere Lage Synthesekautschuk, und dabei insbesondere bevorzugt Nitrilkautschuk eingesetzt.

In einer weiteren bevorzugten Ausführung umfassen die erste Lage und die zweite Lage Synthesekautschuke. Diese Ausführungsform ist insbesondere für Anwender mit Naturlatexallergie, aber auch bei Behandlung von Patienten mit Naturlatexallergie vorgesehen. Eine besonders vorteilhafte Ausführungsform des Handschuhs umfasst in der ersten inneren Lage synthetisches Polyisopren und in der zweiten äußeren Lage Nitrilkautschuk.

Der Einsatz von Naturkautschuk oder synthetischem Polyisopren als erste innere Lage ist vorteilhaft, da hiermit eine hochelastische und dennoch reißfeste Lage, welche die Grundstruktur des Handschuhs bildet, bereitgestellt wird. Der Einsatz von Nitrilkautschuk als zweite äußere Lage ist vorteilhaft, da hiermit ein Polymer Verwendung findet, welches im polymerisierten Zustand weniger flexibel ist als polymerisierter Naturkautschuk bzw. polymerisiertes synthetisches Polyisopren. Der vordere Abschnitt ist dabei insgesamt weniger flexibel und bietet damit die erwünschte Schutzfunktion unter Beibehalt der flexiblen Stulpe.

Als besonderes vorteilhaft erweist sich, wenn die erste Lage und die zweite Lage eine unterschiedliche Farbe aufweisen. Durch die unterschiedlichen Farben der ersten und zweiten Lage werden dem Anwender das Vorhandsein eines vorderen Abschnittes und eines hinteren Abschnittes und insbesondere damit das Vorhandensein des Verstärkungsbereiches vermittelt. Als für den Anwender besonders vorteilhaft ist, wenn der Verstärkungsbereich, und insbesondere dessen definierte Eigenschaften für den Anwender unmittelbar über eine Farbcodierung erkennbar sind.

Weiter vorzugsweise umfasst der medizinische Handschuh weitere an die erste Lage nach innen angrenzende Lagen.

Insbesondere ist eine weitere innere Lage vorgesehen, die die Gleitfähigkeit der Innenseite und damit die Anziehbarkeit des medizinischen Handschuhs verbessert.

Damit ist insbesondere vorgesehen, dass der medizinische Handschuh innen puderfrei ist.

Diese weitere Lage kann insbesondere geformt sein aus einem Acrylsäure-basierendem Polymer, aus Polyurethan-basierendem

Polymer, aus Silikon-basierendem Polymer oder durch eine Hydrogel-basierende Beschichtung.

Weiter vorzugsweise beschichtet diese weitere innere Lage die erste innere Lage nur bereichsweise, derart, dass der hintere Abschnitt über die Länge von vorzugsweise mindestens 5 mm, insbesondere mindestens 15 mm, weiter insbesondere mindestens 25 mm und höchstens 40 mm gemessen vom offenen Ende unbeschichtet verbleibt. Der unbeschichtete Bereich ist vorteilhaft für die bessere Haftfähigkeit und damit für ein vermindertes Aufrollen des Handschuhs. Diese weitere innere Lage umfasst vorzugsweise eine Mischung aus Polyurethan und Polyacrylaten und/oder Derivaten.

Der wegwerfbare, medizinische Handschuh erfüllt je nach Anwendungsbereich die nach EN 455-2:2000 vorgeschriebenen Abmaße hinsichtlich Mindestlänge und Breite. Die Bestimmung der Länge und Breite erfolgt nach der darin beschriebenen Methode.

Der erfindungsgemäße medizinische Handschuh ist vorzugsweise ein Untersuchungshandschuh oder ein Pflegehandschuh oder ein Operationshandschuh. Vorzugsweise ist der medizinische Handschuh ein steriler Operationshandschuh.

Ebenso erfindungsgemäß ist die Herstellung eines wegwerfbaren mehrlagigen medizinischen Handschuhs in einer definierten Schrittabfolge eines Tauchverfahrens umfassend die folgenden Schritte:

a) Bereitstellen eines handähnlichen Formwerkzeugs umfassend einen Fingerbereich, einen Handflächenbereich und einen Stulpenbereich in der Orientierung der Fingerkuppen nach unten

b) Eintauchen des Formwerkzeugs in Tauchtiefe T1a in eine Dispersion mit Koagulantmittel von erster Konzentration K1, wobei die Tauchtiefe T1a mindestens der Gesamtlänge L des herzustellenden Handschuhs entspricht.

c) Eintauchen des Formwerkzeugs in Tauchtiefe T2a in eine Dispersion mit Koagulantmittel von zweiter Konzentration K2, wobei die zweite Konzentration K2 geringer als die erste Konzentration K1 ist und wobei die Tauchtiefe T2a geringer als die Tauchtiefe T1a ist.

d) Eintauchen des Formwerkzeugs in Tauchtiefe T2b in eine zweite Polymerdispersion P2 zur Bildung der zweiten äußeren Lage des Handschuhs, wobei die Tauchtiefe T2b geringer ist als die Tauchtiefe T1a ist.

e) Eintauchen des Formwerkzeugs in Tauchtiefe T1b in eine erste Polymerdispersion P1 zur Bildung der ersten inneren Lage des Handschuhs, wobei die Tauchtiefe T1b größer als die Tauchtiefe T2b ist.

Als Formwerkzeug kann dabei eine Form aus jeglichem für das Tauchverfahren geeigneten Material eingesetzt werden.

Das handähnliche Formwerkzeug umfasst dabei einen Fingerbereich, einen Handflächenbereich und einen Stulpenbereich, mit den für den herzustellenden Handschuh gewünschten Dimensionen, insbesondere als anatomisches Formwerkzeug für die Herstellung von Operationshandschuhen.

Das Formwerkzeug wird in der Orientierung der Fingerkuppen nach unten in eine Dispersion mit Koagulantmittel von erster Konzentration K1 eingetaucht.

Als Koagulantmittel werden vorzugsweise zweiwertige kationische Metallsalze eingesetzt, insbesondere Calciumnitrat, Calciumchlorid, Zinknitrat, Zinkchlorid, Magnesiumacetat, Magnesiumnitrat oder Kombinationen davon, und weiter vorzugsweise werden dreiwertige kationische Metallsalze eingesetzt, insbesondere Aluminiumnitrat, Aluminiumsulfat oder Kombinationen davon.

Mittels der Konzentration der Koagulantmittel lässt sich die Dicke des herzustellenden polymeren Gegenstandes bzw. die Dicke von Bereichen des herzustellenden polymeren Gegenstandes steuern. Koagulantmittel höherer Konzentration führen zu größeren Wanddicken, hingegen werden Koagulantmittel niedriger Konzentration für die Herstellung von geringeren Wanddicken eingesetzt.

Bekannterweise ist die Dicke des herzustellenden polymeren Gegenstandes bzw. die Dicke eines Bereiches des herzustellenden polymeren Gegenstandes zusätzlich über die Verweildauer in der jeweiligen Polymerdispersion und auch deren Konzentration einstellbar.

Die Tauchtiefe T1a für Überziehen des Formwerkzeugs mit einem Koagulantmittel mit Konzentration K1 entspricht dabei mindestens, insbesondere annähernd der Gesamtlänge L des herzustellenden medizinischen Handschuhs.

Das aus Schritt b) mit einem Überzug an Koagulantmittel K1 erhaltene Formwerkzeug wird im Schritt c) in eine Dispersion von Koagulantmittel von geringerer zweiter Konzentration K2 mit der Tauchtiefe T2a eingetaucht. Die Tauchtiefe T2a ist dabei geringer als die Tauchtiefe T1a. Das Koagulantmittel erster Konzentration K1 wird über die Erstreckung der Tauchtiefe T2a mit dem Koagulantmittel geringerer zweiter Konzentration K2 modifiziert.

Die Tauchtiefe T2a entspricht dabei der Längserstreckung L2 der zweiten äußeren Lage des im herzustellenden medizinischen Handschuh vorgesehenen vorderen Abschnitts, wobei der vordere Abschnitt den Fingerbereich vollständig und den Handflächenbereich zumindest bereichsweise, vorzugsweise ebenfalls vollständig enthält.

Das Formwerkzeug aus Schritt c), das auf seiner Oberfläche mit Kogulantmittel erster Konzentration K1 und mit Koagulantmittel zweiter Konzentration K2 umgeben ist, wird im Schritt d) in eine zweite Polymerdispersion P2 mit der Tauchtiefe T2b eingetaucht, wobei die Tauchtiefe T2b geringer ist als die Tauchtiefe T1a. Die zweite Polymerdispersion P2 dient zur Bildung der zweiten äußeren Lage des medizinischen Handschuhs.

Vorzugsweise entspricht die Tauchtiefe T2b annähernd der Tauchtiefe T2a, mit einem Abweichungsspielraum von höchstens ± 10 % bezogen auf die Tauchtiefe T2a.

Die Tauchtiefe T2b zur Bildung der zweiten äußeren Lage entspricht dabei annähernd der Längserstreckung L2 der zweiten äußeren Lage des im herzustellenden medizinischen Handschuh vorgesehenen vorderen Abschnitts, wobei der vordere Abschnitt den Fingerbereich vollständig und den Handflächenbereich zumindest bereichsweise, vorzugsweise ebenfalls vollständig enthält.

Das aus Schritt d) mit der zweiten Polymerdispersion P2 überzogene Formwerkzeug wird im Schritt e) in eine erste Polymerdispersion P1 mit der Tauchtiefe T1b eingetaucht, wobei die Tauchtiefe T1b größer ist als die Tauchtiefe T2b. Die erste Polymerdispersion P1 dient zur Bildung der ersten inneren Lage des medizinischen Handschuhs.

Vorzugsweise entspricht die Tauchtiefe T1b annähernd der Tauchtiefe T1a, mit einem Abweichungsspielraum von höchstens ±5 % bezogen auf die Tauchtiefe T1a.

Vorzugsweise entspricht die Tauchtiefe T1b zur Bildung der ersten inneren Lage annähernd der Gesamtlänge L des herzustellenden medizinischen Handschuhs.

Der am Formwerkzeug gebildete und dieses Formwerkzeug umgebende polymere Handschuh wird unter Wenden vom Formwerkzeug, also unter Inversion des Stulpenbereichs, des Handflächenbereichs und des Fingerbereichs abzogen, so dass die zweite Lage die zweite äußere Lage des Handschuhes in dem dem Anwender angebotenen und bereitgestellten Zustand bildet.

Weiter vorteilhaft wird vor dem Schritt e) das Formwerkzeug in Tauchtiefe T2c in eine Lösung mit einer oberflächenaktiven Komponente eingetaucht wird, wobei die Tauchtiefe T2c geringer als die Tauchtiefe T1a ist.

Vorzugsweise entspricht die Tauchtiefe T2c annähernd der Tauchtiefe T2b, mit einem Abweichungsspielraum von höchstens ± 2 % bezogen auf die Tauchtiefe T2b.

Die Lösung mit einer oberflächenaktiven Komponente aktiviert die Oberfläche der auf dem Formwerkzeug aus Schritt d) hervorgegangenen zweiten äußeren Lage für den Tauchvorgang in Schritt e) zur Herstellung der ersten inneren Lage.

Der Zwischenschritt mit oberflächenaktiven Komponenten ist insbesondere vorteilhaft, um eine vollständige und eine verstärkte Bindungskraft an der Kontaktfläche zwischen zweiter äußerer Lage und erster innerer Lage zu erhalten.

In dem Verfahren wird vorteilhafterweise nach mindestens einem der Schritte d) und e) ein Zwischenschritt mit Trocknen und/oder Härten durchgeführt.

Weiter vorteilhaft wird in dem Verfahren nach Schritt e) das Formwerkzeug in mindestens ein weiteres Tauchbad gegeben zur Bildung von weiteren inneren Lagen. Vorzugsweise wird das Formwerkzeug nach Schritt e) in mindestens eine weitere Polymerdispersion P3 zur Bildung mindestens einer weiteren inneren Lage eingetaucht.

Vorzugsweise enthält die weitere Polymerdispersion P3 Komponenten zur Verbesserung der Gleitfähigkeit der vorgesehenen Innenseite des Handschuhs.

Als Komponenten zur Herstellung einer gleitfähigen Innenseite können dem Fachmann bekannte Materialien eingesetzt werden. Diese Schicht kann geformt sein aus einem Acrylsäure-basierendem Polymer. Weiter kann diese Schicht durch eine Polyurethan-Beschichtung, eine Silikon-Beschichtung oder eine Hydrogel-Beschichtung hergestellt sein.

Weiter vorteilhafterweise wird bei der Beaufschlagung des medizinischen Handschuhs mit der Dispersion P3 die Tauchtiefe T3 gewählt, wobei die Tauchtiefe T3 geringer als die Tauchtiefe T2b ist, um somit im Bereich des offenen Endes des hinteren Abschnittes, also am offenen Ende des Stulpenbereiches einen Bereich mit einer höheren Gleitreibung auf der Innenseite beizubehalten, was für eine bessere Haftung und damit ein vermindertes Aufrollen von Vorteil ist.

In einer weiteren vorteilhaften Ausführung des Verfahrens wird der vom Formwerkzeug unter Inversion der Lagenabfolge abgezogene Handschuh einer separaten Nachbehandlung auf der Innenseite und/oder der Außenseite unterzogen.

Als Nachbehandlung wird vorzugsweise eine Chlorierung der Außenseite zur Bildung einer Oberflächenstrukturierung durchgeführt.

In vorteilhafter Ausführung unterscheiden sich die im Verfahren eingesetzte Polymerdispersion P1 und Polymerdispersion P2 in ihren polymeren Materialien.

Als polymere Materialien können die Materialien wie vorangehend beschrieben eingesetzt werden.

Besonders bevorzugt umfasst die Polymerdispersion P1 Naturkautschuk, weiter insbesondere synthetisches

Polyisopren. Die Polymerdispersion P2 umfasst vorzugsweise Nitrilkautschuk.

Weitere Merkmale, Vorteile und Einzelheiten der Erfindung ergeben sich aus den beigefügten Patentansprüchen und aus der zeichnerischen Darstellung und nachfolgenden Beschreibung bevorzugter Ausführungsformen der Erfindung.

Die Erfindung soll im Folgenden anhand von Figuren näher erläutert werden, dabei zeigen

Figur 1: schematisch einen erfindungsgemäßen medizinischen Handschuh,

Figur 2: schematisch den Lagenaufbau des medizinischen Handschuhs aus Figur 1 im Querschnitt Q -- Q,

Figur 3: die Messpunkte für die Messung der Dicke eines Handschuhs nach ISO 10282:2002,

Figur 4: schematisch ein erfindungsgemäßes Herstellungsverfahren für einen bereichsweise mehrlagigen medizinischen Handschuh.

Figur 5: die Nadelspitze für die Messung der Durchstichfestigkeit in Anlehnung an die ASTM D 3787-01

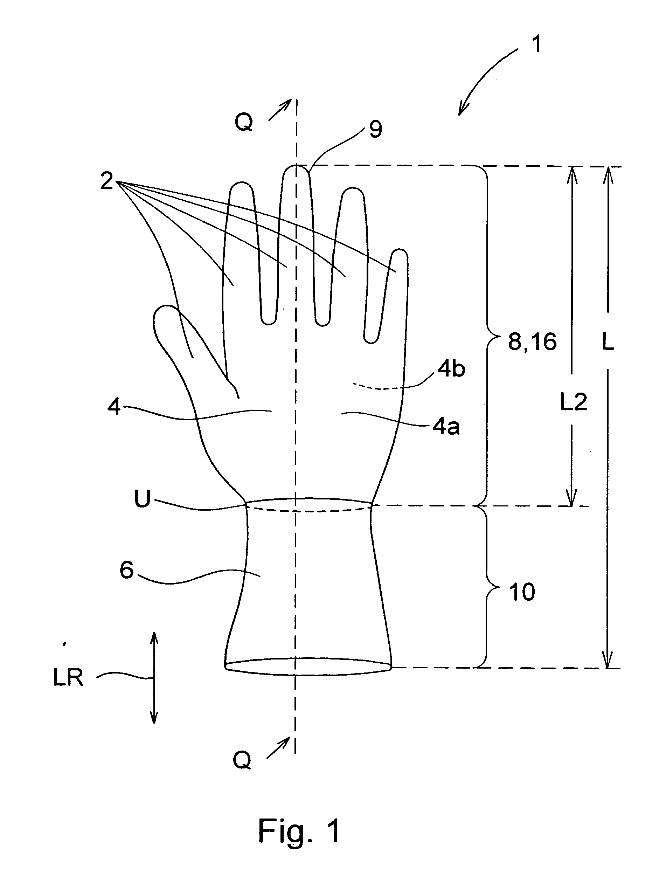

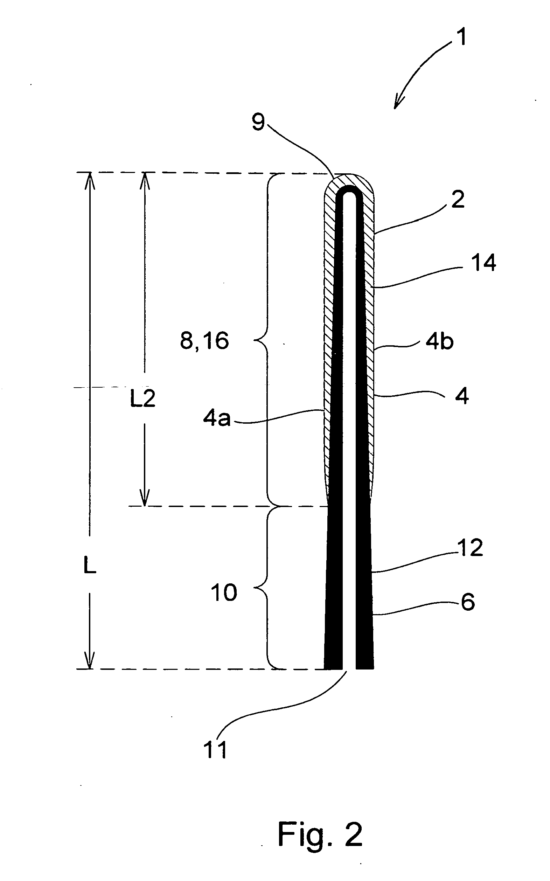

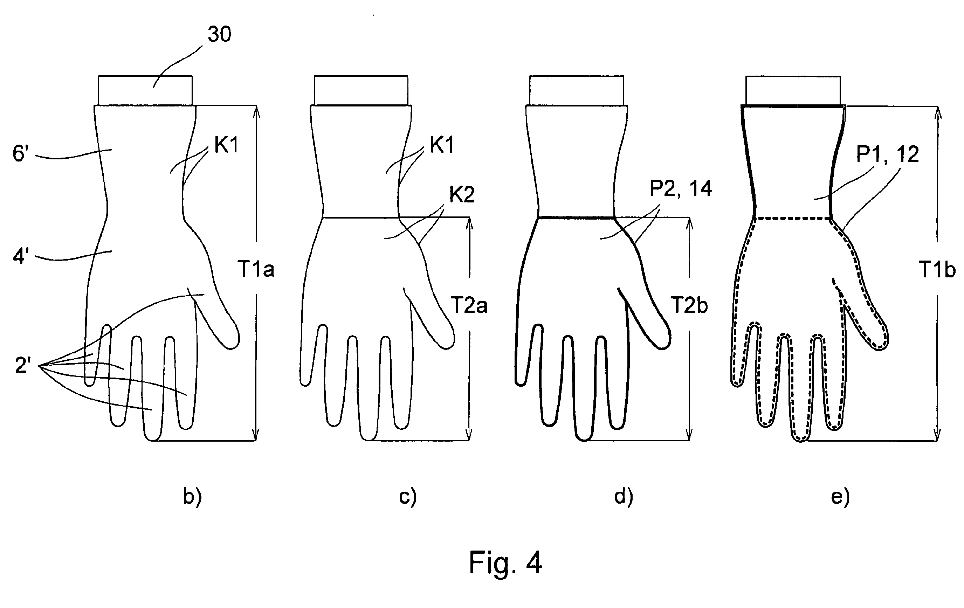

Figur 1 und Figur 2 im Längsschnitt entlang der Linie Q-Q zeigen schematisch einen wegwerfbaren, medizinischen Handschuh in Form eines anatomisch geformten Operationshandschuhs 1 mit einem Fingerbereich 2, einem Handflächenbereich 4 und einem Stulpenbereich 6.
Der Stulpenbereich 6 beginnt in dem Bereich, an dem der Handflächenbereich 4 beim Übergang in den Stulpenbereich 6 den geringsten Umfang U aufweist.
Der Handflächenbereich umfasst die Handinnenfläche 4a und den Handrücken 4b. Der Operationshandschuh 1 hat einen vorderen Abschnitt 8 mit einem geschlossenen Ende 9, welches durch die Fingerkuppen 9 der einzelnen Fingeraufnahmebereiche gebildet wird, und einen daran anschließenden hinteren Abschnitt 10 mit einem offenen Ende 11, welches durch die herkömmliche Anziehöffnung des Handschuhs im Stulpenbereich 6 gebildet ist. Der vordere Abschnitt 8 enthält den Fingerbereich 2 und den Handflächenbereich 4 vollständig. Der hintere Abschnitt 10 enthält den Stulpenbereich 6. Der vordere Abschnitt 8 wird gebildet und begrenzt durch den Verstärkungsbereich 16. Der Verstärkungsbereich 16 umfasst dabei eine erste innere Lage 12 und eine zweite äußere Lage 14, der hintere Abschnitt 10 nur die erste innere Lage 12, wie der Querschnitt durch den Handschuh entlang Q-Q in Figur 2 zeigt.
Die erste innere Lage 12 umfasst als polymeres Material Naturkautschuk. Die zweite äußere Lage 14 umfasst als polymeres Material Nitrilkautschuk. Die erste innere Lage 12 erstreckt sich über den vorderen Abschnitt 8 beginnend vom geschlossenen Ende, also den Fingerkuppen bis zum offenen Ende, also der Anziehöffnung, des hinteren Abschnitts 10. Diese erste innere Lage weist in dieser Richtung über diese Gesamtlänge L des medizinischen Handschuhs ein zunehmendes Dickenprofil auf. Die an die innere Lage angrenzende zweite äußere Lage 14 erstreckt sich über eine Länge L2 ausgehend vom geschlossenen Ende des vorderen Abschnitts über den Fingerbereich 2 und den Handflächenbereich 4 vollständig und endet an der Grenze zwischen Handflächenbereich 4 und Stulpenbereich 6. Die Erstreckung der äußeren Lage 14 und damit des Verstärkungsbereiches 16 fällt also im dargestellten Fall mit der Erstreckung des Fingerbereichs 2 und des Handflächenbereiches 4 zusammen.
Denkbar wäre auch, die äußere Lage 14 nur über einen Teil des Handflächenbereiches 4 zu erstrecken oder die äußere Lage 14 über den Handflächenbereich 4 hinaus in den Stulpenbereich 6 hinein zu erstrecken.
Die zweite äußere Lage 14 weist dabei in dieser Richtung über die Längserstreckung L2 des vorderen Abschnitts ein abfallendes Dickenprofil auf.
Insgesamt hat der Operationshandschuh 1 über seine Gesamtlänge L beginnend vom geschlossenen Ende 9 in Längsrichtung LR zum offenen Ende 11 eine Gesamtdicke pro Einzelwand mit einem abfallenden Dickenprofil, das heißt die Dicke nimmt in dieser Richtung ab.
Die zweite Lage ist zusätzlich mit einem Farbstoff/Pigment versetzt, so dass der Anwender den durch die erste innere Lage und die zweite äußere Lage gebildeten Verstärkungsbereich 16 durch die Farbgebung der äußeren zweiten Lage vom hinterem Abschnitt 10, hier also dem Stulpenbereich 6 unterscheiden kann.
Der Operationshandschuh weist dabei in den entsprechenden Messpunkten gemäß ISO 10282:2002, wie in Figur 3 schematisch wiedergegeben ist, folgende Gesamtdicke auf: Der Operationshandschuh hat im Stulpenbereich, also bei Messpunkt 3 eine Gesamtdicke von 0,23 - 0,27 mm, im Handflächenbereich, also bei Messpunkt 2 eine Gesamtdicke von 0,25 - 0,28 mm und im Fingerbereich, also bei Messpunkt 1 eine Gesamtdicke von 0,28 - 0,34 mm.
Die Gesamtdicke ist derart, dass der Anwender mit angezogenem Handschuh trotz Verstärkungsbereich weiterhin ein ausreichendes Tastgefühl hat und der Tragekomfort ohne schnelle Ermüdung der Hände möglich ist.
Der Operationshandschuh ist zusätzlich mit einer weiteren inneren Beschichtung (nicht dargestellt) zur Verbesserung der Gleitfähigkeit ausgestattet. Diese Innenbeschichtung umfasst Polyurethan und/oder Polyacrylate und/oder Derivate.
Diese Schicht ermöglicht ein leichteres Anziehen des Operationshandschuhs mit dem Vorteil, dass der Operationshandschuh auf der Innenseite puderfrei ist.
Der Operationshandschuh hat im Verstärkungsbereich 16 eine höhere Durchstichfestigkeit als im hinteren Abschnitt 10.
In einer weiteren, nicht dargestellten Ausführungsform der Erfindung umfasst der in Figur 1 und Figur 2 dargestellte Operationshandschuh 1 eine erste innere Lage 12 aus synthetischem Polyisopren und eine zweite äußere Lage 14 aus Nitrilkautschuk.
Der Operationshandschuh nach Figur 1 und Figur 2 wird dabei nach dem Tauchverfahren, wie schematisch in Figur 4 dargestellt, hergestellt.
Nach Bereitstellung eines einer Hand anatomisch nachgeformten Formwerkzeuges 30 mit einem Fingerbereich 2', einem Handflächenbereich 4' und einem Stulpenbereich 6' wird dieses Formwerkzeug mit den Fingerkuppen nach unten in Schritt b) in ein Koagulant mit erster Konzentration K1 eingetaucht, und zwar in der Tauchtiefe T1a, welche mindestens der Erstreckung der Gesamtlänge L des herzustellenden Operationshandschuhs 1 entspricht, also den Fingerbereich 2, den Handflächenbereich 4 und den Stulpenbereich 6 umfasst.

Im nachfolgenden Schritt c) wird das bereichsweise mit Koagulantmittel K1 umgebene Formwerkzeug in ein Koagulantbad mit Koagulantmittel mit im Vergleich zu K1 geringeren zweiten Konzentration K2 eingetaucht, wobei die dabei vorgenommene Tauchtiefe T2a so tief ist, dass der Fingerbereich vollständig und auch der Handflächenbereich annähernd vollständig mit Koagulantmittel K2 umgeben werden.

Nachfolgend wird in Schritt d) das mit Koagulantmittel K1 und Koagulantmittel K2 umgebene Formwerkzeug mit der Tauchtiefe T2b in eine Polymerdispersion P2 eingetaucht. Die Tauchtiefe T2b entspricht dabei annähernd der Tauchtiefe T2a, so dass auch hier der Fingerbereich und der Handflächenbereich vollständig mit der Polymerdispersion P2 umgeben sind.

Als Polymerdispersion P2 ist eine Acrylnitril-Butadienhaltige Zusammensetzung eingesetzt, zur Bildung der zweiten äußeren Lage 14 aus Nitrilkautschuk.

Das aus nach Schritt d) erhaltende Formwerkzeug, dessen Fingerbereich und Handflächenbereich an der Oberseite nun mit der Acrylnitril-Butadien-haltigen Zusammensetzung umgeben ist und dessen Stulpenbereich weiterhin mit dem Koagulantmittel K2 umgeben ist, wird in einem nachfolgenden Schritt in eine Polymerdispersion P1 eingetaucht, und zwar in der Tauchtiefe T1b. Die Tauchtiefe T1b entspricht der Erstreckung des Operationshandschuhs mit der Gesamtlänge L, also die Tauchtiefe T1b entspricht annähernd der Tauchtiefe T1a.

Als Polymerdispersion P1 ist eine Naturlatex-haltige Zusammensetzung eingesetzt, zur Bildung der inneren ersten Lage 12.

In einer bevorzugten alternativen Ausführungsform ist als Polymerdispersion P1 eine Zusammensetzung enthaltend synthetisches Polyisopren eingesetzt.

Nach Trocknen und Aushärten der inneren ersten Lage 12 und der äußeren zweiten Lage 14 wird das Formwerkzeug in eine weitere Zusammensetzung enthaltend Gleitreibung reduzierende Substanzen eingetaucht (nicht dargestellt), und zwar in einer Tauchtiefe, die geringer als die Gesamtlänge des Operationshandschuhs ist, so dass die innere erste Lage im Randbereich unterhalb des offenen Endes des Stulpenbereichs unbeschichtet bleibt.

Als Gleitreibung reduzierende Substanz wird vorzugsweise eine Mischung aus Polyurethan und Polyacrylaten eingesetzt.

**Patentansprüche**

1. Wegwerfbarer mehrlagiger medizinischer Handschuh (1) aus polymerem Material, wobei der medizinische Handschuh ein Untersuchungshandschuh oder ein Operationshandschuh ist, mit einem Fingerbereich (2), einem Handflächenbereich (4) und einem Stulpenbereich (6), mit einem vorderen Abschnitt(8) mit einem geschlossenen Ende (9) und einem hinteren Abschnitt (10) mit offenem Ende (11), wobei der vordere Abschnitt (8) den Fingerbereich (2) vollständig und den Handflächenbereich (4) zumindest bereichsweise enthält, und wobei eine erste innere polymere Lage (12) den vorderen Abschnitt (8) und den hinteren Abschnitt (10) umfasst und wobei die erste innere polymere Lage (12) in dem vorderen Abschnitt (8) mit einer zweiten äußeren polymeren Lage (14)zur Bildung eines Verstärkungsbereiches (16) beschichtet ist, so dass die erste und die zweite Lage dort eine polymere zweilagige Verbundstruktur bilden, und wobei der Verstärkungsbereich (16) den vorderen Abschnitt (8) bildet und begrenzt, wobei die äußere Lage im für den Gebrauch vorgesehenen und im für den Anwender bereitgestellten Zustand und auch im angezogenen Zustand des Handschuhs eine dem Körper des Anwenders abgewandte Lage ist, wobei der vordere Abschnitt (8) und der hintere Abschnitt (10) sich in der Gesamtdicke unterscheiden,
**dadurch gekennzeichnet, dass**
die Gesamtdicke im Stulpenbereich (6) geringer ist als im Handflächenbereich(4) und/oder geringer als im Fingerbereich (2), mit der Gesamtdicke gemessen an den Messpunkten gemäß ISO 10282:2002 (E),
dass die erste innere Lage (12) ausgehend vom geschlossenen Ende(9) des vorderen Abschnitts (8) in Längsrichtung LR zum offenen Ende (11) des hinteren Abschnitts (10) eine Dicke mit einem ansteigenden Dickenprofil aufweist und dass die zweite äußere Lage (14) beginnend vom geschlossenen Ende (9) des vorderen Abschnitts (8) in Längsrichtung LR innerhalb des vorderen Abschnitts eine Dicke mit einem abfallenden Dickenprofil aufweist.

2. Wegwerfbarer mehrlagiger medizinischer Handschuh nach Anspruch 1, **dadurch gekennzeichnet, dass** der vordere Abschnitt(8)den Handflächenbereich(4)vollständig enthält.

3. Wegwerfbarer mehrlagiger medizinischer Handschuh nach Anspruch 1 oder 2, **dadurch gekennzeichnet, dass** der vordere Abschnitt (8) und der hintere Abschnitt (10) sich unterscheiden in den polymeren Materialien und/oder in den physikalischen Eigenschaften, davon insbesondere entnommen aus der Gruppe Reißkraft, Reißfestigkeit, Reißdehnung, maximaler Kraftaufwand oder maximale Spannung bei 300% Dehnung, maximaler Kraftaufwand oder maximale Spannung bei 500% Dehnung, Durchstichfestigkeit und/oder in der Barrierewirkung und/oder in der Farbe.

4. Wegwerfbarer mehrlagiger medizinischer Handschuh nach einem der vorstehenden Ansprüche, **dadurch gekennzeichnet, dass** die Gesamtdicke im Stulpenbereich (6) zur Gesamtdicke im Handflächenbereich (4)in einem Verhältnis von höchstens 1: 1,2 vorliegt, mit der Gesamtdicke gemessen an den Messpunkten gemäß ISO 10282:2002 (E).

5. Wegwerfbarer mehrlagiger medizinischer Handschuh nach einem der vorstehenden Ansprüche, **dadurch gekennzeichnet, dass** der Handschuh über die Gesamtlänge L ausgehend vom geschlossenen Ende(9)des vorderen Abschnitts(8)eine Gesamtdicke mit einem abfallenden Dickenprofil aufweist.

6. Wegwerfbarer mehrlagiger medizinischer Handschuh nach Anspruch 5 mit einer Gesamtdicke von höchstens 0,40 mm, vorzugsweise höchstens 0,35 mm, weiter vorzugsweise höchstens 0,30 mm im vorderen Abschnitt und einer Gesamtdicke von mindestens 0,15 mm, vorzugsweise mindestens 0,18 mm, weiter vorzugsweise mindestens 0,20 mm im hinteren Abschnitt, mit der Dicke gemessen gemäß ISO 4648:1991, Methode A.

7. Wegwerfbarer mehrlagiger medizinischer Handschuh nach einem der vorstehenden Ansprüche, **dadurch gekennzeichnet, dass** der vordere Abschnitt (8) bei 300% Dehnung eine höheren maximalen Kraftaufwand aufweist als der hintere Abschnitt.

8. Wegwerfbarer mehrlagiger medizinischer Handschuh nach einem der vorstehenden Ansprüche, **dadurch gekennzeichnet, dass** der vordere Abschnitt (8) bei 500% Dehnung eine höhere Spannung aufweist als der hintere Abschnitt.

9. Wegwerfbarer mehrlagiger medizinischer Handschuh nach einem der vorstehenden Ansprüche, **dadurch gekennzeichnet, dass** der vordere Abschnitt (8) eine größere Durchstichfestigkeit aufweist als der hintere Abschnitt.

10. Wegwerfbarer mehrlagiger medizinischer Handschuh nach einem der vorstehenden Ansprüche, **dadurch gekennzeichnet, dass** die erste innere Lage (12) und/oder zweite äußere Lage(14) polymere Materialien umfasst, welche entnommen sind aus der Gruppe Naturkautschuk und synthetischer Kautschuk, davon insbesondere synthetisches Polyisopren, Polychloropren, Acrylonitril-Butadien-Kautschuk, oder Kombinationen dieser polymeren Materialien.

11. Medizinischer Handschuh nach Anspruch 10, **dadurch gekennzeichnet**, das die erste innere Lage (12) Naturkautschuk oder synthetisches Polyisopren und die zweite äußere Lage (14) Acrylonitril-Butadien-Kautschuk umfasst.

12. Medizinischer Handschuh nach einem der vorstehenden Ansprüche, **dadurch gekennzeichnet, dass** der Stulpenbereich(6)eine Gesamtdicke von 0,23 - 0,27 mm aufweist, der Handflächenbereich(4)eine Gesamtdicke von 0,25 - 0,28 mm aufweist und der Fingerbereich (2)eine Dicke von 0,28 - 0,34 mm aufweist, mit der Gesamtdicke gemessen an den Messpunkten gemäß ISO 10282:2002 (E).

13. Verfahren zum Herstellen eines wegwerfbaren mehrlagigen medizinischen Handschuhs nach Anspruch 1 umfassend die Schrittabfolge:

a) Bereitstellen eines handähnlichen Formwerkzeugs (30) umfassend einen Fingerbereich (2'), einen Handflächenbereich (4') und einen Stulpenbereich (6') in der Orientierung der Fingerkuppen nach unten
b) Eintauchen des Formwerkzeugs in volle Tauchtiefe T1a für die Herstellung eines Handschuhs mit der Gesamtlänge L in eine Dispersion mit Koagulantmittel von erster Konzentration K1, wobei die Tauchtiefe T1a mindestens der Gesamtlänge L des herzustellenden Handschuhs entspricht
c) Eintauchen des Formwerkzeugs in Tauchtiefe T2a in eine Dispersion mit Koagulantmittel von zweiter Konzentration K2, wobei die zweite Konzentration K2 geringer als Konzentration K1 ist und wobei die Tauchtiefe T2a geringer als Tauchtiefe T1a ist
gekennzeichnet über die nachfolgenden Schritte
d) Eintauchen des Formwerkzeugs in Tauchtiefe T2b in eine zweite Polymerdispersion P2 zur Bildung der zweiten äußeren polymeren Lage (14), wobei die Tauchtiefe T2b geringer als die Tauchtiefe T1a ist
e) Eintauchen des Formwerkzeugs in Tauchtiefe T1b in eine erste Polymerdispersion P1 zur Bildung der ersten inneren polymeren Lage(12), wobei die Tauchtiefe T1b größer als die Tauchtiefe T2b ist,

und wobei der nach den vorstehenden Schritten am Formwerkzeug gebildete und dieses Formwerkzeug umgebende polymere Handschuh unter Wenden vom Formwerkzeug abzogen wird, also unter Inversion des Stulpenbereichs, des Handflächenbereichs und des Fingerbereichs, so dass die zweite Lage(14) die zweite äußere Lage (14) des Handschuhes in dem dem Anwender angebotenen und bereitgestellten Zustands bildet.

14. Verfahren nach Anspruch 13, wobei Tauchtiefe T2a und/oder Tauchtiefe T2b der Längserstreckung L2 der zweiten äußeren Lage des herzustellenden medizinischen Handschuhs entspricht.

15. Verfahren nach Anspruch 13 oder 14, wobei vor dem Schritt e) das Formwerkzeug in Tauchtiefe T2c in eine Lösung mit einer oberflächenaktiven Komponente eingetaucht wird, wobei die Tauchtiefe T2c geringer als die Tauchtiefe T1a ist.

16. Verfahren nach Anspruch 13 oder 15, wobei nach mindestens einem der Schritte d) und e) ein Zwischenschritt Trocknen und/oder Härten durchgeführt wird.

## Claims

1. A disposable multilayer medical glove (1) made of polymer material, which medical glove is an examination glove or a surgical glove having a finger area (2), a hand surface area (4), and a cuff area (6) having a front section (8) with a closed end (9) and having a rear section (10) with an open end (11), wherein the front section (8) entirely contains the finger area (2) and at least partly contains the hand surface area (4), wherein a first inner polymer layer (12) comprises the front section (8) and the rear section (10), and wherein the first inner polymer layer (12) is coated in the front section (8) with a second outer polymer layer (14) to form a reinforcement area (16), so that the first and second layer form a two-layer polymer composite structure, and wherein the reinforcement area (16) forms and delimits the front section (8), wherein the outer layer forms a layer facing away from the body of the user when it is in a conformation designed for use and ready for the user, and also when it has been pulled on, wherein the front section (8) and the rear section (10) differ in total thickness,
   **characterized in that**
   the total thickness in the cuff area (6) is less than in the hand surface area (4) and/or less than in the finger area (2), with the total thickness being measured at the measurement points according to ISO 10282:2002 (E),
   the first inner layer (12) has a thickness that increases in profile from the closed end (9) of the front section (8) in a longitudinal direction LD to the open end (11) of the rear section (10), and
   the second outer layer (14) has a thickness that decreases in profile from the closed end (9) of the front section (8) in a longitudinal direction LD within the front section.

2. Disposable multilayer medical glove according to Claim 1, **characterized in that** the front section (8) entirely contains the hand surface area (4).

3. Disposable multilayer medical glove according to Claim 1 or 2, **characterized in that** the front section (8) and the rear section (10) differ in polymer materials and/or physical properties, taken in particular from the group composed of tearing strength, tear resistance, elongation at break, required maximum force, or maximum tension at 300% elongation, maximum force required or maximum tension at 500% elongation, puncture resistance, and/or in the barrier effect, and/or the color.

4. Disposable multilayer medical glove according to one of the aforementioned claims, **characterized in that** the maximum ratio of the total thickness in the cuff area (6) to the total thickness in the hand surface area (4) is a maximum of 1 : 1.2, with the total thickness being measured at the measurement points according to ISO 10282: 2002 (E).

5. Disposable multilayer medical glove according to one of the aforementioned claims, **characterized in that** the glove shows a total thickness that decreases in profile over the full length L from the closed end (9) of the front section (8).

6. Disposable multilayer medical glove according to Claim 5 having a total maximum thickness of 0.40 mm, preferably a maximum of 0.35 mm, and even more preferably a maximum of 0.30 mm in the front section and a total thickness of at least 0.15 mm, preferably at least 0.18 mm, and even more preferably at least 0.20 mm in the rear section, with the thickness being measured at the measurement points according to ISO 4648:1991, Method A.

7. Disposable multilayer medical glove according to one of the aforementioned claims, **characterized in that** the front section (8) shows a higher required maximum force than the rear section at elongation of 300%.

8. Disposable multilayer medical glove according to one of the aforementioned claims, **characterized in that** the front section (8) shows higher tension than the rear section at elongation of 500%.

9. Disposable multilayer medical glove according to one of the aforementioned claims, **characterized in that** the front section (8) shows greater puncture resistance than the rear section.

10. Disposable multilayer medical glove according to one of the aforementioned claims, **characterized in that** the first inner layer (12) and/or second outer layer (14) comprise polymer materials from the group composed of natural rubber and synthetic rubber, and among these, particularly synthetic polyisoprene, polychloroprene, acrylonitrile-butadiene-rubber, or combinations of these polymer materials.

11. Medical glove according to Claim 10, **characterized in that** the first inner layer (12) comprises natural rubber and the second outer layer (14) comprises acrylonitrile-butadiene-rubber.

12. Medical glove according to one of the aforementioned claims, **characterized in that** the cuff area (6) has a total thickness of 0.23-0.27 mm, the hand surface area (4) has a total thickness of 0.25-0.28 mm, and the finger area (2) has a thickness of 0.28-0.34 mm, with the total thickness being measured at the measurement points according to ISO 10282:2002 (E).

13. Process for the production of a disposable multilayer medical glove according to Claim 1, comprising the following series of steps:

   a) preparation of a hand-like mould (30) comprising a finger area (2'), a hand surface area (4'), and a cuff area (6') with the fingertips facing downward;
   b) immersion of the mould to the full immersion depth T1a for the production of a glove with a total length L in a dispersion with a coagulant having a first concentration C1, wherein the immersion depth T1a is at least equivalent to the total length L of the glove to be produced;
   c) immersion of the mould to the immersion depth T2a in a dispersion with a coagulant having a second concentration C2, wherein the second concentration C2 is less than concentration C1 and wherein the immersion depth T2a is less than the immersion depth T1a;
   **characterized by** the following steps:
   d) immersion of the mould to the immersion depth T2b in a second polymer dispersion P2 in order to form the second outer polymer layer (14), wherein the immersion depth T2b is less than the immersion depth T1a.
   e) immersion of the mould to the immersion depth T1b in a first polymer dispersion P1 in order to form the first inner polymer layer (12), wherein the immersion depth T1b is greater than the immersion depth T2b,
   and wherein the polymer glove formed on the mould after the aforementioned steps and surrounding said mould is pulled off the mould with a turning motion, i.e., with the cuff area, the hand surface area, and the finger area being inverted, so that the second layer (14) forms the second outer layer (14) of the glove in the configuration provided for the user and ready for use.

14. Process according to Claim 13, wherein the immersion depth T2a and/or the immersion depth T2b corresponds to the longitudinal length L2 of the second outer layer of the medical glove to be produced.

15. Process according to Claim 13 or 14, wherein, before step e), the mould is immersed to the immersion depth T2c in a solution with a surfactant component, wherein the immersion depth T2c is less than the immersion depth T1a.

16. Process according to Claim 13 or 15, wherein an intermediate step of drying and/or curing is carried out after at least one of steps d) and e).

**Revendications**

1. Gant médical multicouche jetable (1) à base de matériau polymère, le gant médical étant un gant pour examen ou un gant chirurgical, comportant une zone de doigts (2), une zone de plat de la main (4) et une zone de manchette (6), comportant une partie avant (8) présentant une extrémité fermée (9) et une partie arrière (10) à extrémité ouverte (11), la partie avant (8) comportant entièrement la zone de doigts (2) et au moins en partie la zone de plat de la main (4), et une première couche polymère interne (12) englobant la partie avant (8) et la partie arrière (10) et la première couche polymère interne (12) étant revêtue d'une deuxième couche polymère externe (14) dans la partie avant (8) pour la formation d'une zone de renforcement (16), de sorte que la première et la deuxième couche y forment une structure composite bicouche polymère, et la zone de renforcement (16) constituant et limitant la partie avant (8), la couche externe en l'état prévu pour l'emploi et en l'état fourni à l'utilisateur et également en l'état porté du gant étant une couche opposée au corps de l'utilisateur, la partie avant (8) et la partie arrière (10) différant quant à l'épaisseur totale,

**caractérisé en ce que**

l'épaisseur totale dans la zone de manchette (6) est plus faible que dans la zone de plat de la main (4) et/ou plus faible que dans la zone de doigts (2), l'épaisseur totale étant mesurée au niveau des points de mesure selon ISO 10282:2002 (E),

**en ce que** la première couche interne (12) présente en partant de l'extrémité fermée (9) de la partie avant (8) dans le sens de la longueur LR à l'extrémité ouverte (11) de la partie arrière (10) une épaisseur présentant un profil d'épaisseur croissant et

**en ce que** la deuxième couche externe (14) présente en commençant de l'extrémité fermée (9) de la partie avant (8) dans le sens de la longueur LR à l'intérieur de la partie avant une épaisseur présentant un profil d'épaisseur décroissant.

2. Gant médical multicouche jetable selon la revendication 1, **caractérisé en ce que** la partie avant (8) contient entièrement la zone de plat de la main (4).

3. Gant médical multicouche jetable selon la revendication 1 ou 2, **caractérisé en ce que** la partie avant (8) et la partie arrière (10) se distinguent quant aux matériaux polymères et/ou aux propriétés physiques, dont en particulier celles prises dans le groupe constitué par la force de rupture, la résistance à la rupture, l'allongement à la rupture, le déploiement de force maximum ou la tension maximale à allongement de 300 %, le déploiement de force maximum ou la tension maximale à allongement de 500 %, la résistance à la perforation et/ou quant à l'effet barrière et/ou quant à la couleur.

4. Gant médical multicouche jetable selon l'une quelconque des revendications précédentes, **caractérisé en ce que** l'épaisseur totale dans la zone de manchette (6) par rapport à l'épaisseur totale dans la zone de plat de la main (4) se trouve en un rapport d'au maximum 1:1,2, l'épaisseur totale étant mesurée au niveau des points de mesure selon ISO 10282:2002 (E).

5. Gant médical multicouche jetable selon l'une quelconque des revendications précédentes, **caractérisé en ce que** sur la longueur totale L partant de l'extrémité fermée (9) de la partie avant (8) le gant présente une épaisseur totale présentant un profil d'épaisseur décroissant.

6. Gant médical multicouche jetable selon la revendication 5, ayant une épaisseur totale d'au maximum 0,40 mm, de préférence d'au maximum 0,35 mm, encore mieux d'au maximum 0,30 mm dans la partie avant et une épaisseur totale d'au moins 0,15 mm, de préférence d'au moins 0,18 mm, encore mieux d'au moins 0,20 mm dans la partie arrière, l'épaisseur totale étant mesurée selon ISO 4648:1991, méthode A.

7. Gant médical multicouche jetable selon l'une quelconque des revendications précédentes, **caractérisé en ce qu'**à un allongement de 300 % la partie avant (8) présente un plus grand déploiement de force maximum que la partie arrière.

8. Gant médical multicouche jetable selon l'une quelconque des revendications précédentes, **caractérisé en ce qu'**à un allongement de 500 % la partie avant (8) présente une plus grande tension que la partie arrière.

9. Gant médical multicouche jetable selon l'une quelconque des revendications précédentes, **caractérisé en ce que** la partie avant (8) présente une plus grande résistance à la perforation que la partie arrière.

10. Gant médical multicouche jetable selon l'une quelconque des revendications précédentes, **caractérisé en ce que** la première couche interne (12) et/ou la deuxième couche externe (14) comprend/comprennent des matériaux polymères qui sont pris dans le groupe constitué par le caoutchouc naturel et le caoutchouc synthétique, dont en particulier le polyisoprène synthétique, le polychloroprène, le caoutchouc acrylonitrile-butadiène, ou des associations de ces matériaux polymères.

**11.** Gant médical selon la revendication 10, **caractérisé en ce que** la première couche interne (12) comprend du caoutchouc naturel ou du polyisoprène synthétique et la deuxième couche externe (14) comprend du caoutchouc acrylonitrile-butadiène.

**12.** Gant médical selon l'une quelconque des revendications précédentes, **caractérisé en ce que** la zone de manchette (6) présente une épaisseur totale de 0,23 - 0,27 mm, la zone de plat de la main (4) présente une épaisseur totale de 0,25 - 0,28 mm et la zone de doigts (2) présente une épaisseur de 0,28 - 0,34 mm, l'épaisseur totale étant mesurée au niveau des points de mesure selon ISO 10282:2002 (E).

**13.** Procédé pour la fabrication d'un gant médical multicouche jetable selon la revendication 1, comprenant la succession d'étapes :

a) fourniture d'un outil de mise en forme (30) ressemblant à une main, comprenant une zone de doigts (2'), une zone de plat de la main (4') et une zone de manchette (6') dans le sens des bouts des doigts vers le bas

b) immersion de l'outil de mise en forme en toute la profondeur d'immersion T1a pour la production d'un gant ayant une longueur totale L, dans une dispersion avec un coagulant à une première concentration K1, la profondeur d'immersion T1a correspondant à au moins la longueur totale L du gant à fabriquer

c) immersion de l'outil de mise en forme en profondeur d'immersion T2a dans une dispersion avec un coagulant à une deuxième concentration K2, la deuxième concentration K2 étant inférieure à la concentration K1 et la profondeur d'immersion T2a étant plus faible que la profondeur d'immersion T1a

**caractérisé par** les étapes subséquentes

d) immersion de l'outil de mise en forme en profondeur d'immersion T2b dans une deuxième dispersion de polymère P2 pour la formation de la deuxième couche polymère externe (14), la profondeur d'immersion T2b étant plus faible que la profondeur d'immersion T1a

e) immersion de l'outil de mise en forme en profondeur d'immersion T1b dans une première dispersion de polymère P1 pour la formation de la première couche polymère interne (12), la profondeur d'immersion T1b étant plus grande que la profondeur d'immersion T2b,

et dans lequel on détache le gant polymère formé après les étapes précédentes sur l'outil de mise en forme et entourant cet outil de mise en forme, en le retournant, c'est-à-dire avec inversion de la zone de manchette, de la zone de plat de la main et de la zone de doigts, de sorte qu'en l'état fourni et offert à l'utilisateur la deuxième couche (14) constitue la deuxième couche externe (14) du gant.

**14.** Procédé selon la revendication 13, dans lequel la profondeur d'immersion T2a et/ou la profondeur d'immersion T2b correspond(ent) à l'extension longitudinale L2 de la deuxième couche externe du gant médical à fabriquer.

**15.** Procédé selon la revendication 13 ou 14, dans lequel avant l'étape e) on immerge l'outil de mise en forme en profondeur d'immersion T2c dans une solution comportant un composant tensioactif, la profondeur d'immersion T2c étant plus faible que la profondeur d'immersion T1a.

**16.** Procédé selon la revendication 13 ou 15, dans lequel après au moins l'une des étapes d) et e) on effectue une étape intermédiaire de séchage et/ou de durcissement.

Q ↗

9

1

2

4b

4a

8,16

L

L2

4

U

6

10

LR

Q ↗

## Fig. 1

Fig. 2

Fig. 3

Fig. 4

## Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 2270618 A
- DE 102005009826 A1
- EP 0561651 A1
- US 5459880 A
- US 20020133864 A1
- BE 654896
- WO 03057444 A1
- US 20030124354 A1